# EUROPEAN PATENT APPLICATION

(11) **EP 2 508 616 A1**
(43) Date of publication of application: **10.10.2012**
(21) Application number: 10834671.9
(22) Date of filing: 03.12.2010
(51) Int. Cl.: C12Q 1/37, A61K 8/96, A61Q 19/00, G01N 33/50, G01N 33/53

(54) **METHOD FOR SCREENING OF SUBSTANCE CAPABLE OF PROMOTING RECOVERY OF SKIN BARRIER FUNCTION**

(30) Priority: 04.12.2009 JP 2009277034
(71) Applicant: Shiseido Company, Ltd., Chuo-ku Tokyo 104-8010 (JP)
(72) Inventor: HIBINO, Toshihiko, Yokohama-shi Kanagawa 236-8643 (JP); YAMAMOTO, Mami, Yokohama-shi Kanagawa 236-8643 (JP)
(74) Representative: Henkel, Breuer & Partner
(86) International application number: PCT/JP2010/071736
(87) International publication number: WO 2011/068221

(57) **Abstract**

A method for screening for a substance capable of promoting the recovery of a skin barrier function, the method comprising the steps of: incubating caspase-14 and prosaposin in the presence of a candidate substance for the substance capable of promoting the recovery of a skin barrier function; and selecting the candidate substance as the substance capable of promoting the recovery of a skin barrier function when the decomposition of prosaposin into saposin A is promoted significantly compared with that achieved in the presence of a reference substance.

## Description

### TECHNICAL FIELD

The present invention relates to a screening method for a substance capable of promoting recovery of skin barrier function, using the degradation of prosaposin by caspase-14 as an indicator.

### BACKGROUND ART

Terminal differentiation of keratinocytes results in the formation of a protective barrier against a harmful environment, referred to as the "stratum corneum". The stratum corneum intercellular lipids play an important role in this protective barrier. The stratum corneum intercellular lipids are biosynthesized in conjunction with the differentiation in epidermal keratinocytes, and are accumulated in the Odland bodies. The sugar chains of glucosylceramide released between stratum corneum cells by exocytosis of the Odland bodies are cleaved with glucocerebrosidase to synthesize ceramide which is the main component of the stratum corneum intercellular lipids. Further, as a separate pathway, the degradation of sphingomyelin into ceramide by sphingomyelinase is known.

The sphingolipid activator protein, saposin, is an indispensable protein in the hydrolysis of sphingolipid, and the four types of saposin (saposin A, saposin B, saposin C, and saposin D) are known to exist. Saposin is a factor functioning as a coenzyme for glucocerebrosidase, sphingomyelinase, and other ceramide synthases, which are necessary in the synthesis of stratum corneum lipids. Thus, saposin seems to be important to a skin barrier function.

To date, it has been verified that the storage of saposin A, C, or D in the internal organs causes lysosomal storage diseases (for example, Gaucher's disease and Newman-Pick Disease), and mutations in the prosaposin gene cause a neuropathic phenotype. In association with the skin, it has been reported that prosaposin is reduced in patients with atopic dermatitis and psoriasis (refer to Tezuka et al., Journal of Investigative Dermatology (1997) 109, 319-323 and Alessandrini et al., Journal of Investigative Dermatology (2001) 116, 394-400, respectively).

It has been known that the loss of moisture from the skin is higher in rough skin pathologies seen in various skin diseases including atopic dermatitis, psoriasis, contact dermatitis, etc., than in healthy skin. It has been thought that an increase of the so-called transepidermal water loss (TEWL) is associated with the reduction of the components which seem to have a role in the retention of moisture and a function as a barrier within the skin.

Until now, it has been reported that the skin function declines as the skin barrier function declines, resulting in abnormal proliferation of the skin, etc. Specifically, in the case of older persons, the recovery of the declined skin barrier function requires a long time, thus, an effective and novel agent for recovery of skin barrier function has been demanded to prevent the abnormal proliferation of the skin, etc., due to the decline of the skin function with aging.

Regarding a substance, which is studied for a formulation for promoting recovery of skin barrier and other cosmetics comprising the substance, the effect of the substance must be verified in tests on animals. However, recently, in the field of cosmetic product development, there is the trend for suppressing tests on animals from the viewpoint of ethics, and the demand for a screening method for candidate substances without applying the substances to animals has been increasing.

### [Prior Art Documents]

### [Non-Patent Documents]

[Non-Patent document 1] Tezuka et al., Journal of Investigative Dermatology (1997) 109, 319-323
[Non-Patent document 2] Alessandrini et al., Journal of Investigative Dermatology (2001) 116, 394-400

### SUMMARY OF THE INVENTION

The problem to be solved by the present invention is to provide a screening method for a substance for recovery of skin barrier function, using a novel indicator.

Caspase-14 is the newest member of the caspase family, and is expressed nearly exclusively in differentiated keratinocytes. Caspase-14 has been identified as EST homologous to caspase family members. According to the findings of recent research, the caspase-14 present in keratinocytes is processed to heterodimers that demonstrate enzymatic activity for Trp-Glu-His-Asp-AFC corresponding to the synthetic substrate for caspase-1 (Mikolajczyk J. et al., Biochemistry 43, 10560-9 (2004)). This hydrolysis activity requires proteolytic cleavage and the presence of a kosmotropic salt. The primary structure of caspase-14 is very similar to those of inflammatory caspases, such as caspase-1, -4 and -5, but the caspase-14 expression confined mainly to differentiated keratinocytes suggests that the expression may be involved in keratinocyte terminal differentiation in a different mode (Lippens S. et al., Cell Death Differ. 7, 1218-24 (2000)). However, the activation mechanism of caspase-14, its natural substrate, and regulatory factors have yet to be elucidated.

Accordingly, the present inventors isolated the proteins capable of binding to caspase-14, from extracts of differentiated and cultured normal human epidermal keratinocytes (NHEK), etc., to subject them to LC/MS/MS analysis. As a result, prosaposin was identified from among the proteins capable of binding to caspase-14.

The present inventors have discovered that prosaposin is expressed in from the basal layer to the granular layer, saposin A is expressed in the uppermost layer of the granular layer, and caspase-14 is involved in the processing of prosaposin to produce an intermediate of saposin and saposin A, and have completed the present invention.

Therefore, the present application includes the following inventions:
(1) A screening method for a substance capable of promoting recovery of skin barrier function, comprising the steps of: incubating caspase-14 and prosaposin in the presence of a candidate substance for the substance capable of promoting recovery of skin barrier function; and selecting the candidate substance as the substance capable of promoting recovery of skin barrier function when the degradation of prosaposin into saposin A is promoted significantly compared to a control substance.
(2) A method according to (1), wherein said degradation is evaluated by Western blotting using an anti-saposin A antibody.

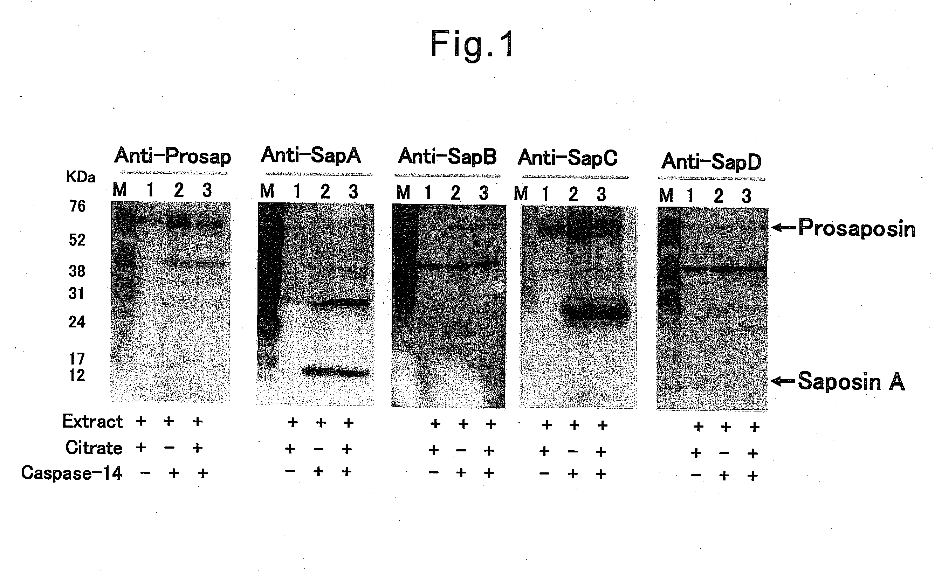

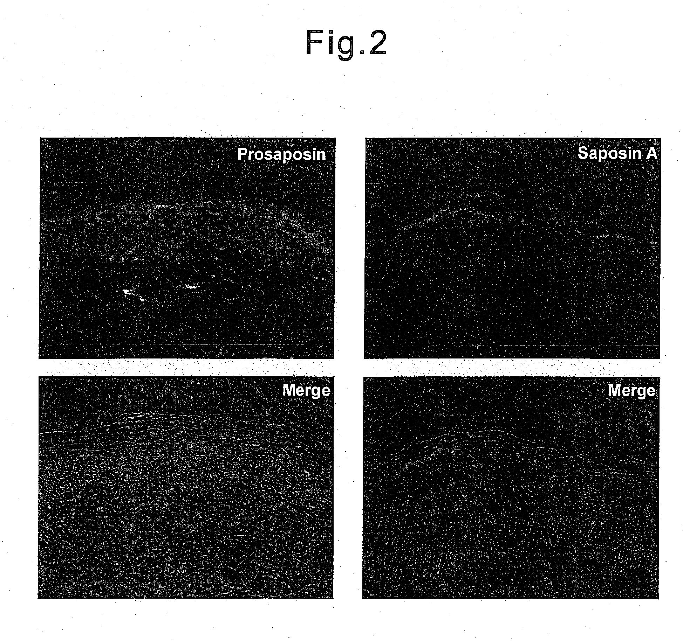

Conventionally, in *in vitro* testing, it has been known that caspase-14 exhibits an activating action only in the presence of a high concentration of kosmotropic salt, for example, citric acid. As a result, the function of c-aspase-14 *in vivo* has been unknown for a long time. At this time, the present inventors have discovered for the first time that, without adding a kosmotropic salt, caspase-14 restrictively decomposes prosaposin to produce an intermediate of saposin and saposin A (FIG. 1). Furthermore, the present inventors have discovered using immunostaining that prosaposin is expressed in from the basal layer to the granular layer and saposin A is present in the upper granular layer of the skin (FIG. 2). From the results mentioned above, it is thought that caspase-14 plays a role in the processing of prosaposin *in vivo.*

Saposin, which is a degradation product of prosaposin, acts as a cofactor for ceramide synthase necessary in the synthesis of stratum corneum intercellular lipid, and is necessary in epidermal barrier formation. Thus, according to the method of the present invention, the search for a novel substance capable of promoting recovery of skin barrier function is possible using the degradation into saposin A by caspase-14 as an indicator without performing testing on animals.

### [Brief Description of the Drawings]

FIG. 1 is a Western blot chart showing the processing of prosaposin in the presence or absence of caspase-14.
FIG. 2 is an immunostaining showing the localization of prosaposin and saposin A in normal human skin.

### DESCRIPTION OF THE PREFERRED EMBODIMENTS

The present invention provides a screening method for a substance capable of promoting recovery of skin barrier function, comprising the steps of incubating caspase-14 and prosaposin in the presence of a candidate substance for the substance capable of promoting recovery of skin barrier function, and selecting the candidate substance as the substance capable of promoting recovery of skin barrier function when the degradation of prosaposin into saposin A is promoted significantly compared to a control substance.

The candidate substance used in the method of the present invention is not limited, but includes, for example, a substance that activates caspase-14 to thereby promote the degradation of prosaposin. Since caspase-14 is a cysteine protease, the candidate substance may be, for example, a reducing agent or a substance having a reduction action, for example, a reagent such as mercaptoethanol and dithiothreitol (DTT); and a biological material such as glutathione; and a plant extract having a reduction action. If the degradation of prosaposin into saposin A is promoted significantly compared to a control substance when caspase-14 and prosaposin are incubated in the presence of the candidate substance, the candidate substance may be selected as the substance capable of promoting recovery of skin barrier function. It may be judged that "the degradation of prosaposin into saposin A is promoted significantly" based on the evaluation criterion, for example, whether the degradation of prosaposin into saposin A is promoted by 10% or more, 20% or more, 30% or more, 50% or more, 70% or more, or 100% compared to the case of the action of the control.

The degradation of prosaposin into saposin A may be evaluated by Western blotting using an anti-saposin A antibody. It is preferable that the antibody is an anti-saposin A specific antibody. However, the present invention is not limited to the means.

A drug selected by the screening method of the present invention is believed to be effective in improving skin in need of recovery of skin barrier function, for example, skin having reduced skin barrier function due to skin diseases, various stresses, and rough skin, etc., grafted skin having a reduced skin barrier function due to the insufficient formation of the skin barrier, and skin having a reduced skin barrier function due to grafting.

The term "skin barrier function" as used herein means the moisture retention, the prevention of infiltration of viruses, bacteria, etc., and the like, by the skin, specifically, the epidermis. The function can be evaluated by measuring the transepidermal water loss (TEWL) (unit: g/m²·h) under the conditions where sweating does not occur. Therefore, according to the present invention, a drug selected as a substance capable of promoting recovery of skin barrier function is applied to the skin, and TEWL is measured to determine whether or not the drug actually recovers the skin barrier function.

Another embodiment provides a method for promoting recovery of skin barrier function, comprising activating caspase-14 to promote the degradation of prosaposin into saposin A.

The method for promoting recovery of skin barrier function of the present invention is preferably provided as a cosmetic method. However, this method is not limited to cosmetic methods, but may be performed for the purpose of treatment depending on the symptoms of the skin in which the recovery of the skin barrier function is intended. The method of the present invention may be carried out by applying the substance for activating caspase-14, for example, a reducing agent, to patients in need of recovery of function. The application method is not specifically limited. For example, a cosmetic containing the substance as the effective ingredient may be applied to the skin in need of recovery of skin barrier function, or, a pack or a sheet comprising the substance may be placed or adhered on the skin. The application amount of the substance can be appropriately determined with reference to the symptoms of the skin. Furthermore, this method is not limited to methods for direct application on the skin, but may be an orally administration method depending on the substance for activating caspase-14.

The present invention will be further explained below with examples. Note that, the present invention is not limited thereto.

### EXAMPLES

### 1. Analysis of Factors Interacting with Caspase-14 Method:

1. Caspase-14 was bound to agarose beads having an anti-caspase-14 antibody immobilized thereon, and was allowed to react with a liquid extract from normal human epidermal keratinocytes (NHEK) after induction of differentiation, in which the aerial exposure and the addition of 2 mM calcium after growth phase and confluency were performed to continue the culture. After washing with PBS, binding protein was eluted by a Glycine-HCl buffer solution (pH 2.3). Further, agarose beads having an anti-caspase-14 antibody immobilized thereon were allowed to react with a liquid extract from normal epidermal stratum corneum and a liquid extract from psoriasis patient's scales. After washing with PBS, binding protein was similarly eluted by a Glycine-HCl buffer solution (pH 2.3). These four samples were analyzed by LC/MS/MS.

### LC/MS/MS Analysis

A silica frit was produced to maintain the filler in the end of the tapered opening of an unfilled capillary column (manufactured by New Objective, Inc.) having an inner diameter of 100 µm and a length of 120 mm. The obtained capillary column was filled with octadecylsilanized silica-type filler Aqua C18 (manufactured by Phenomenex, Inc.) having an average particle size of 5 µm until the height reached 100 mm, to obtain a reversed-phase capillary column for analysis.

A silica frit was produced to maintain the filler in the end of the opening of an unfilled capillary column (manufactured by Agilent) having an inner diameter of 250 µm and a length of 150 mm. The obtained capillary column on the outlet side was filled with a mixture of a cation-exchange resin type filler Partisphere SCX resin (manufactured by Whatman, Inc.) having An average particle size of 5 µm and an anion-exchange resin type filler PolyWAXLP (manufactured by PolyLC, Inc.) having an average particle size of 5 µm at a mass ratio of 2:1 until the height reached 25 mm. The obtained capillary column on the inlet side was filled with octadecylsilanized silica type filler Aqua C18 (manufactured by Phenomenex, Inc.) having an average particle size of 5 µm until the height reached 25 mm. A two-phase capillary column consisting of a reversed-phase capillary column for traps and a SCX-WAX mixed capillary column was obtained.

Upon the production of the reversed-phase capillary column for analysis and the two-phase type capillary column, the filler was filled by a slurry filling method using a high pressure nitrogen gas and a pressure-type filling container.

Next, the aforementioned four samples were analyzed by the six-step MudPIT type analysis (two dimensional HPLC/ESI MS/MS).

First, a supernatant containing approximately 4 µg of peptide was loaded in the two-phase type capillary column by a pressure method. Thereafter, cleaning and desalting were carried out using a mobile phase A (a mixed liquid having a volume ratio of water, acetonitrile, and formic acid of 95:5:0.1; pH approximately 2.6) having a volume 10 times or more of the sample solution. The two-phase type capillary column 10 was connected with the reversed-phase capillary column for analysis 20 via a through hole type union (manufactured by Upchurch Scientific, Inc) (not shown). Next, a Nanospace SI-2 type HPLC device (manufactured by Shiseido Co., Ltd.) using a capillary having an inner diameter of 100 µm as pipework was connected therewith. At the time, the reversed-phase capillary column for traps 11 was disposed upstream of the SCX-WAX mixed capillary column 12 and the reversed-phase capillary column for analysis 20.

As the mobile phases, mobile phase A, mobile phase B (a mixed liquid of a volume ratio of water, acetonitrile, and formic acid was 20:80:0.1), and mobile phase C (mobile phase A containing 500 mM ammonium acetate; pH approximately 6.8) were used. The elution method of the peptide was a gradient elution method comprising a total of six steps, in which the vol% of mobile phase C added in a rectangular shape was gradually increased at each step.

The gradient profile of Step 1 was as follows: The mobile phase A was applied for five minutes, the rate of the mobile phase B was increased from 0 vol% to 15 vol% over the next five minutes, the rate of the mobile phase B was increased to 45 vol% over the next 60 minutes, and the rate of the mobile phase B was increased to 75 vol% over the next ten minutes. Thereafter, the flow rate was maintained at this rate for five minutes.

The gradient profiles of Steps 2 to 6 were as follows: The mobile phase A was applied for one minute, the rate of the mobile phase C was flown for four minutes as X [vol%], the rate of the mobile phase C was increased from 0 vol% to 15 vol% over the next five minutes, the rate of the mobile phase C was increased to 45 vol% over the next 60 minutes, and the rate of the mobile phase C was increased to 75 vol% over the next ten minutes. Thereafter, the flow rate was maintained at this rate for five minutes. At the time, the flow rate of a solution sent from a pump was set to 250 µL/min, and the flow rate for the column was adjusted to 300 to 400 nL/min by splitting using a resistance type capillary.

Further, in the ESI MS/MS measurement, an ion trap mass spectrometer LCQ-Deca (manufactured by ThermoFisher Scientific, Inc.) was used. At the time, the peptide eluted from the reversed-phase capillary column for analysis was directly introduced into a mass spectrometer without splitting.

Note that, a cycle of one full scan MS spectrum measurement at a mass-to-charge ratio (m/z) of 400 to 1400 and three data-dependent MS/MS spectrum measurements was repeated in the respective steps. The normalized collision energy was 35%. Three microscans were performed for each of the MS spectrum measurement and the MS/MS measurement. The dynamic exclusion settings were a repeat count of 1, a repeat duration of 0.50 min, an exclusion list size of 25, and an exclusion duration of 10.00 min.

The obtained MS/MS spectra were searched in the non-redundant human database (ftp://ftp.ncbi.nih.gov/blast/db/FASTA.nr.gz, 2007/2/8 version) using the SEQUEST algorithm operating on Bioworks Software (manufactured by Thermo Fisher Scientific, Inc.)

The results of analysis revealed the presence of prosaposin was common only in the reaction sample with NHEK after induction of differentiation and the sample after the reaction with the liquid extract from normal epidermal stratum corneum.

### 2. Processing of Prosaposin by Caspase-14

Prosaposin is not processed and exists as a precursor in a monolayer culture of normal keratinocytes. Using this, the reaction of prosaposin in a precursor state with caspase-14 was examined. A prosaposin-containing liquid extraction from normal human epidermal keratinocytes (NHEK) was allowed to react with activated caspase-14 at 37°C for one hour in the presence or absence of 1.2 M of kosmotropic ions (citric acid ions). After dialysis in water, freeze-drying was performed. This was dissolved in an SDS electrophoresis sample buffer. After SDS electrophoresis, the production of each saposin was examined by Western blotting. The antibodies used respectively were an anti-prosaposin antibody (PSAP polyclonal antibody (A01): Abnova Corporation), an anti-Saposin A specific antibody (manufactured in-house), an anti-Saposin B antibody (E-15, sc-27014: Santa Cruz Biotechnology, Inc.), an anti-Saposin C antibody (H-81,sc-32875: Santa Cruz Biotechnology, Inc.), and an anti-Saposin D antibody (E-14, sc-27024R: Santa Cruz Biotechnology, Inc.) The dilution ratio used was 1/1000 in each case. Note that, all the antibodies except for the anti-Saposin A specific antibody recognized prosaposin. The results are shown in FIG. 1.

From the results in FIG. 1, it is understood that prosaposin degraded into a plurality of intermediates in the presence of caspase-14. Thereamong, saposin A, which is one of the final degradation products of prosaposin, was recognized by the anti-Saposin A specific antibody, thus, it is thought that caspase-14 plays a role in degrading prosaposin into saposin A.

### 3. Immunostaining

Immunostaining was performed to clarify the localization of prosaposin and saposin A using a normal skin section obtained by plastic surgery. A paraffin section was deparaffinized with xylene and was returned to a PBS solution. Thereafter, blocking was carried out with 10% goat serum. After PBS washing, a reaction with an anti-prosaposin antibody (PSAP mouse polyclonal antibody (A01): Abnova Corporation, dilution ratio: 1/200) or an anti-saposin A specific antibody (manufactured in-house) (dilution ratio: 1/200) was carried out at room temperature for one hour. After washing three times with PBS, a reaction with AlexaFluor 488 goat anti-mouse IgG (prosaposin) or AlexaFluor555 goat anti-rabbit IgG (saposin A) was carried out at room temperature for one hour. After washing three times with PBS, it was covered with a Vector Shield mounting medium (DAPI) (Vector Laboratories, Inc.) to perform observation by a fluorescence microscope. The results are shown in FIG.2.

From the results in FIG. 2, it is understood that prosaposin was present in from the basal layer to the granular layer of normal human epidermis, and saposin A was expressed in the uppermost layer of the granular layer. These results as well as the results of the aforementioned Western blotting have supported that caspase exclusively expressed in keratinocytes degrades prosaposin in human skin, and plays an important role in skin barrier function.

## Claims

1. A screening method for a substance capable of promoting recovery of skin barrier function, comprising the steps of: incubating caspase-14 and prosaposin in the presence of a candidate substance for the substance capable of promoting recovery of skin barrier function, and
selecting the candidate substance as the substance capable of promoting recovery of skin barrier function when the degradation of prosaposin into saposin A is promoted significantly compared to a control substance.

2. A method according to claim 1, wherein said degradation is evaluated by Western blotting using an anti-saposin A antibody.

3. A method for promoting recovery of skin barrier function, comprising activating caspase-14 to promote the degradation of prosaposin into saposin A.

4. A method according to claim 3, comprising applying to the skin a substance for activating caspase-14.
